# EUROPEAN PATENT SPECIFICATION

(11) **EP 4 633 730 B1**
(45) Date of publication and mention of the grant of the patent: **25.11.2026**
(21) Application number: 23822379.6
(22) Date of filing: 13.12.2023
(51) Int. Cl.: A61N 1/372, A61B 5/00, A61N 1/378, H02J 7/00, H02J 50/00, H04B 5/00

(54) **METHODS FOR ALIGNING AN ACCESSORY DEVICE WITH AN ELECTRONIC IMPLANT, A RELATED ACCESSORY DEVICE AND A RELATED ELECTRONIC IMPLANT**
VERFAHREN ZUR AUSRICHTUNG EINER ZUBEHÖRVORRICHTUNG MIT EINEM ELEKTRONISCHEN IMPLANTAT, ZUGEHÖRIGE ZUBEHÖRVORRICHTUNG UND ZUGEHÖRIGES ELEKTRONISCHES IMPLANTAT
PROCÉDÉS D'ALIGNEMENT D'UN DISPOSITIF ACCESSOIRE AVEC UN IMPLANT ÉLECTRONIQUE, DISPOSITIF ACCESSOIRE ASSOCIÉ ET IMPLANT ÉLECTRONIQUE ASSOCIÉ

(30) Priority: 16.12.2022 LU 503204
(43) Date of publication of application: 22.10.2025
(73) Proprietor: PRECISIS GmbH, 69124 Heidelberg (DE)
(72) Inventor: ABKAI, Ciamak, 68542 Heddesheim (DE); JAGSCHIES, Lasse, 69118 Heidelberg (DE); TITTELBACH, Michael, 69126 Heidelberg (DE)
(74) Representative: Aera A/S
(86) International application number: PCT/EP2023/085566
(87) International publication number: WO 2024/126574

(56) References cited:
- US-A1- 2013 096 651
- US-A1- 2017 361 116
- US-A1- 2019 151 668
- US-A1- 2022 203 103

## Description

The present disclosure pertains to the field of electronic implants, such as implants for neurological stimulation or cardiac stimulation. The present disclosure relates to methods for aligning an accessory device with an electronic implant, a related electronic implant, a related accessory device, and a related wireless interaction system for charging the electronic implant.

### BACKGROUND

Electronic implants, such as neural implants or cardiac implants, are electronic devices that connect to organs of a biological subject to establish a biomedical prosthesis circumventing areas in the organ that have become dysfunctional. The electronic implants may be implanted underneath the skin of the biological subject. Cardiac implants, such as pacemakers, are electronic devices that connect directly to a heart of the biological subject. Neural implants, also referred to as brain implants, are electronic devices configured to stimulate the brain, such as neuronal brain tissue, of the biological subject. Neural implants electrically stimulate, block and/or record signals from single neurons or groups of neurons, such as biological neural networks, in the brain. Depending on the location of the dysfunctional area of the organ, such as an epileptic focus or a seizure focus in the brain, the surgical position of the electronic implant may vary.

To ensure a proper function of the electronic implant, an accessory device may be used for interacting with the electronic implant, such as for communicating with or charging the electronic implant. The quality of the interaction between the electronic implant and the accessory device can be heavily dependent on the relative position and/or orientation of the electronic implant and the accessory device. However, a correct positioning and orientation of the accessory device in relation to the electronic implant can be hard to achieve by a user due to the non-visible position of the electronic implant under the skin of the biological subject.

### SUMMARY

Accordingly, there is a need for devices and methods for aligning an accessory device with an electronic implant, which may mitigate, alleviate, or address the shortcomings existing and may provide an improved quality of interaction between the electronic implant and the accessory device.

A method is disclosed, performed in an electronic implant, for aligning an accessory device with the electronic implant. The electronic implant comprises an interface for interacting with the accessory device, processing circuitry, and an electromagnetic positioning coil. The method comprises monitoring a presence of the accessory device. The method comprises, upon determining that the accessory device is present, activating the electromagnetic positioning coil to generate an electromagnetic field for aligning the accessory device.

Further, an electronic implant is disclosed, the electronic implant comprising an interface for interacting with an accessory device, an electromagnetic positioning coil, processor circuitry, and an energy storage. The electronic implant is configured to perform the any of the methods disclosed herein.

Further, a computer readable storage medium is disclosed. The computer readable storage medium stores one or more programs, the one or more programs comprising instructions, which when executed by an electronic implant cause the electronic implant to perform any of the methods disclosed herein.

It is an advantage of the present disclosure that the electronic implant can activate and/or deactivate the electromagnetic positioning coil based on whether an accessory device is present. By activating the electromagnetic positioning coil when the accessory device is present a magnetic field can be generated for attracting and aligning a magnetic device in the accessory device. Thereby, the accessory device can be positioned and orientated in relation to the electronic implant, so that an interaction interface, such as a charging interface and/or a communication interface, of the accessory device overlaps with an interaction interface of the electronic implant. By aligning the accessory device so that the interaction interface of the accessory device overlaps with an interaction interface of the electronic implant, the quality of the interaction between the accessory device and the electronic implant can be improved. By activating the electromagnetic positioning coil only when an accessory device is present, the electromagnetic positioning coil does not influence other diagnostics processes, such as Magnetic Resonance Imaging (MRI). Contrary to ferromagnetic parts, the inactive electromagnetic positioning coil does not create any artifacts, such as distortions of the image, in the MRI that could otherwise affect the quality of the MRI exam. Typically, permanent magnets may cause complete image deletion in the MRI due to high susceptibility changes, such as high changes to the extent the magnet is magnetized in an external magnetic field of the MRI scanner. Furthermore, by deactivating the electromagnetic positioning coil when an accessory device is not present a dislocation of the electronic implant due to the influence of magnetic fields, for example during an MRI, can be reduced and/or eliminated. Furthermore, no intervention, such as removal of the electronic implant, is required before performing the MRI scan. Further, the final positioning and alignment can be automated, such as performed by the magnetic field generated by the electromagnetic positioning coil, so that the user is not required to adjust the positioning and alignment by hand based on signaling of information indicative of the positioning of the accessory device between the electronic implant and the accessory device.

A method is disclosed, performed in an accessory device, for aligning the accessory device with an electronic implant. The accessory device comprises an interface for interacting with the accessory device, processing circuitry and a positioning device. The method comprises receiving, from the electronic implant, a signal indicative of an interaction parameter. The method comprises providing an output based on the signal indicative of the interaction parameter.

Further, an accessory device is disclosed. The accessory device comprises processing circuitry, a positioning device, and a wireless interface. The accessory device is configured to perform any of the methods disclosed herein.

Further, a computer readable storage medium is disclosed. The computer readable storage medium storing one or more programs, the one or more programs comprising instructions, which when executed by an accessory device cause the accessory device to perform any of the methods disclosed herein.

It is an advantage of the present disclosure that the accessory device can be informed by the electronic implant about conditions of the interaction between the accessory device and the electronic implant which enables the accessory device to adapt the interaction based on the signal indicative of the interaction parameter received from the electronic implant. The accessory device can for example start and/or adjust a charging of the electronic implant and/or a communication with the electronic implant based on the signal indicative of the interaction parameter to improve the quality of the interaction.

A wireless interaction system for interacting with an electronic implant is disclosed. The wireless charging system comprises the accessory device and the electronic implant disclosed herein.

It is an advantage of the present disclosure that the electronic implant can activate and/or deactivate the electromagnetic positioning coil based on whether an accessory device is present. By activating the electromagnetic positioning coil when the accessory device is present a magnetic field can be generated for attracting and aligning a magnetic device in the accessory device. Thereby, the accessory device can be positioned and orientated in relation to the electronic implant, so that an interaction interface, such as a charging interface and/or a communication interface, of the accessory device overlaps with an interaction interface of the electronic implant. By aligning the accessory device so that the interaction interface of the accessory device overlaps with an interaction interface of the electronic implant, the quality of the interaction between the accessory device and the electronic implant can be improved. By activating the electromagnetic positioning coil only when an accessory device is present, the electromagnetic positioning coil does not influence other diagnostics processes, such as Magnetic Resonance Imaging (MRI). Contrary to ferromagnetic parts, the inactive electromagnetic positioning coil does not create any artifacts, such as distortions of the image, in the MRI that could otherwise affect the quality of the MRI exam. Typically, permanent magnets may cause complete image deletion in the MRI due to high susceptibility changes, such as high changes to the extent the magnet is magnetized in an external magnetic field of the MRI scanner. Furthermore, by deactivating the electromagnetic positioning coil when an accessory device is not present a dislocation of the electronic implant due to the influence of magnetic fields, for example during an MRI, can be reduced and/or eliminated.

Furthermore, no intervention, such as removal of the electronic implant, is required before performing the MRI scan. Documents US 2013/096651 and US 2022/203103 both disclose devices and methods known in the art.

### BRIEF DESCRIPTION OF THE DRAWINGS

The above and other features and advantages of the present disclosure will become readily apparent to those skilled in the art by the following detailed description of examples thereof with reference to the attached drawings, in which:
Fig. 1 is a flow-chart illustrating an example method, performed in an electronic implant, for aligning an accessory device with the electronic implant according to this disclosure,
Fig. 2 is a flow-chart illustrating an example method, performed in an accessory device, for aligning the accessory device with an electronic implant according to this disclosure,
Fig. 3 is a block diagram illustrating an example electronic implant according to this disclosure,
Fig. 4 is a block diagram illustrating an example accessory device according to this disclosure, and
Fig. 5 is a block diagram illustrating an example wireless interaction system comprising the electronic implant and the accessory device according to this disclosure.

### DETAILED DESCRIPTION

Various examples and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the examples. They are not intended as an exhaustive description of the disclosure or as a limitation on the scope of the disclosure. In addition, an illustrated example needs not have all the aspects or advantages shown. An aspect or an advantage described in conjunction with a particular example is not necessarily limited to that example and can be practiced in any other examples even if not so illustrated, or if not so explicitly described.

The figures are schematic and simplified for clarity, and they merely show details which aid understanding the disclosure, while other details have been left out. Throughout, the same reference numerals are used for identical or corresponding parts.

Electronic implants, such as rechargeable electronic implants, can be surgically positioned at different positions under the skin of a biological subject, such as a person. The position of the electronic implant may be selected based on the area to be treated, such as based on an epileptic focus of the biological subject. **In** order to enable optimal interaction between an external accessory device, such as a charging by an external charging unit, a positioning and, if necessary, an orientation of the accessory device in relation to an interaction interface of the electronic implant is relevant for the quality of the interaction between the accessory device and the electronic implant. For example, the positioning and/or the orientation of an external charging device in relation to a charging coil in the electronic implant is relevant for the quality of the coupling and thus for the quality of the energy transfer between the external charging device and the charging coil. The quality of the charging also affects the charging time.

To enable an inductive energy coupling between the charging device and the electronic implant, the charging device must be fixed in the correct place by a user, such as a bearer of the electronic implant. There are various techniques for influencing the location and/or position of the charging device and the receiving unit, such as the charging coil of the electronic implant, in relation to one another.

In the simplest case, the accessory device, such as the charging device, may be fixed in relation to the electronic implant by a mechanical support device arranged on the body of the bearer of the electronic implant, such as fixed to a head of the bearer with one or more of hoods, caps, and bands. However, such a solution may greatly impair the comfort of the patient.

In other known solutions, the implant may send information indicative of a misalignment of the charging device to the charging device, for example in the form of a data message or morse code. This information may then be forwarded to the user, for example as a visual or audible notification. Based on this indication the user of the implant must manually change the position of the charging device until a better alignment is achieved. Such a solution is time consuming and causes annoyance for the user.

Other systems, such as cochlear implants, may use permanent magnets, which due to Lorentz forces fixate the position of the accessory device to the implant, without having to use mechanical fixation means, such as hoods, bands and/or straps. Permanent magnets however have the disadvantage that ferromagnetic material and magnets lead to considerable artifacts in the imaging during an MRI acquisition.

Since the position of electronic implants can vary from bearer to bearer, a bearer-specific charging device is required in order to position the accessory device, such as the charging device, in the correct location. However, the quality of the charging can also be impaired by the use of conventional fixation mean, such as hoods, caps, and/or bands.

The current disclosure provides a solution which provides a high-quality charging of the electronic implant, without generating MRI artifacts during an MRI scan of the bearer of the implant.

An electronic implant, a method performed in the electronic implant, an accessory device, a method performed in the accessory device, and a system comprising the electronic implant and the accessory device is disclosed.

The electronic implant may be a rechargeable electronic implant. The electronic implant comprises an interaction interface, such as a charging interface and/or a communication interface, for interacting with an accessory device, such as a charging device. The electronic implant may comprise one or more electrodes for providing electronic stimulation of tissue, such as brain tissue, of the bearer of the electronic implant. The electronic implant further comprises one or more electromagnetic positioning coil(s), such as a Helmholtz coil, a Maxwell coil, or a Saddle coil, for generating a magnetic field for positioning and/or aligning the accessory device with the electronic implant. The magnetic field for positioning and/or aligning the accessory device may herein be referred to as a magnetic alignment field and/or a magnetic positioning field. The magnetic field for positioning and/or aligning the accessory device is configured to attract and/or position and/or align the accessory device when the accessory device is in the vicinity of the electronic implant. In other words, the electromagnetic positioning coil crates a magnetic field that physically attracts the accessory device, such that the accessory device is pulled towards the electronic implant and into a correct position so that the interaction interfaces of the accessory device and the electronic implant align. In one or more example electronic implants, the electronic implant comprises a plurality of electromagnetic positioning coils, such as a first electromagnetic positioning coil and a second electromagnetic positioning coil. The first and the second electromagnetic positioning coil may be the same type of charging coils or may be different type of positioning coils. In one or more example electronic implants, the first electromagnetic positioning coil is a Helmholtz coil or a Maxwell coil, and the second electromagnetic positioning coil is a Saddle coil. By using a plurality of electromagnetic positioning coils, a rotation of the accessory device in relation to the electronic implant may be controlled in addition to the positioning and orientation. The electronic implant may be one or more of an implant for neurological applications, such as an implant for head based applications, such as an implant for brain stimulation, an implant for cortex stimulation, a cochlear implant, and/or an implant for nerve stimulation, and an implant for chest based applications, such as an implant for cardiac stimulation, such as a pacemaker. In one or more example implants, the implant may comprise a first part being head based and a second part being chest based. The head based part may comprise one or more electrodes for stimulating brain tissue. The chest based part may comprise an energy storage and/or the charging device.

In one or more example electronic implants, the one or more electrodes may be comprised in an electrode pad. The electrode pad may comprise the plurality of electrodes, such as a plurality of electrode surfaces. The electrode pad may be configured to be located in a subgaleal space between a skull and the skin of the bearer of the electronic implant. In other words, the electronic implant may comprise the electrode pad. The electrode pad, such as the electrodes, may be connected to an electronic pulse generator, such as an Implantable Pulse Generator (IPG), by a set of wires. The set of wires may be configured to transfer electric signals and/or currents from the electronic pulse generator to the electrode pad and/or from the electrode pad to the electronic pulse generator. The set of wires can herein be seen as one or more wires. The electronic pulse generator may be located chest-based or head-based and may be realized in one or more housing elements. The electronic pulse generator may be connected to one or more wireless charging interface(s) and one or more electromagnetic positioning coil(s). The one or more wireless charging interface(s) and one or more electromagnetic positioning coil(s) may, in one or more example electronic implants, be arranged outside the housing of the electronic pulse generator, such as in a second housing separate from the housing of the electronic pulse generator. The electronic pulse generator configured to generate electrical stimulation pulses which are transferred by the set of wires to the electrode pad. The electrical stimulation pulses are configured to create, such as generate, therapeutic electrical fields for clinical applications below the skull at the neurological brain tissue of the bearer of the electronic implant.

The components of the electronic implant, such as the interaction interface, the electromagnetic positioning coil, the electrodes, processor circuitry, energy storage, etc., may be comprised in one single housing or may be distributed in different housings, such as in a first housing and a second housing.

The charging interface of the electronic implant may be a first wireless charging interface, such as a receiving charging interface or a secondary charging interface, for generating a current when the first wireless charging coil is interface is exposed to a magnetic field or an electric field, such as a magnetic field or electric field generated by a second wireless charging interface, such as a transmitting charging interface or a primary charging interface, comprised in an accessory device. The first wireless charging interface and the second wireless charging interface may be inductive charging coils or capacitive power transfer (CPT) coupling couplers. The first wireless charging and the second wireless charging are configured to establish a wireless energy transfer from the accessory device to the electronic implant.

The electronic implant may, in one or more examples herein, monitor a presence of the accessory device. Monitoring can herein be seen as observing over time. Monitoring the presence of the accessory device, may in one or more example methods, comprise monitoring a quality of charging, such as monitoring one or more charging parameters generated in the first wireless charging interface, such as in the inductive charging coil or the CPT coupling coupler, of the electronic implant. The one or more charging parameters may be one or more of a current, a voltage, a power, and parameters based on a spectrum of the signal, such as a central frequency and a Q-factor of the signal, generated in the wireless charging interface. The Q-factor is a measure of the quality of coupling factor and can be determined by Q= w_c/(w_1 +w_2), where w_c is a central frequency and w_1 and w_2 are the -3 dB frequencies of the spectrum.

The electronic implant may, in one or more example electronic implants, comprise a monitoring unit, such as processor circuitry, for monitoring the quality of charging. The monitoring unit, such as the processor circuitry, may be configured to monitor the quality of charging, such as the one or more charging parameters, directly on or at the first wireless charging interface, after pre-processing, and/or using digital and/or analogue monitoring solutions. A digital solution is for example that the voltage is discretized by an Analog digital converter (ADC) and is monitored by a Micro-Controller. In the analog solution a transistor may monitor the voltage level, for example on the Base-Emitter-potential. Once a switching voltage level is reached the transistor can be enabled to switch the collector-emitter current.

The accessory device may be determined to be present upon the monitored quality of charging being equal to or above a charging threshold, such as equal to or above a charging current threshold, a charging voltage threshold, a charging power threshold, a charging quality threshold, and/or a spectrum driven threshold, such as a Q-factor threshold.

Upon detecting that the accessory device is present, such as upon the monitored charging quality and/or charging parameters being equal to or above the charging threshold or the magnetic switch being activated, the electronic implant may activate the electromagnetic positioning coil. The electromagnetic positioning coil may be activated by energizing the electromagnetic positioning coil. The electromagnetic positioning coil may be energized using energy stored in an energy storage of the electronic implant or by using a part of the energy generated in the first wireless charging interface, such as in the inductive charging coil or the CPT coupling coupler, of the electronic implant during a charging procedure. The electromagnetic positioning coil may thus only be activated when the accessory device is present, such as in the event of charging. The electromagnetic positioning coil may otherwise not be energized and therefore does not trigger any MRI artifacts during an MRI scan of the bearer of the implant. Since a charging of the implant is not intended during an MRI scan, activation of the electromagnetic positioning coil during charging does not affect the MRI.

In one or more examples herein, so that the electromagnetic positioning coil does not interact with an MRI system in a coupled Radio Frequency (RF) manner, the electromagnetic positioning coil can be configured to be fundamentally detuned, in order not to cause any danger or other artefacts in the MRI. Detuning may be achieved by preventing high frequency oscillation in the electromagnetic positioning coil. Since the electromagnetic positioning coil may mainly be powered in direct current (DC) conditions, parallel in-winding diodes can avoid an alternating current (AC) oscillation. The electromagnetic positioning coil may be detuned either via a winding of the coil or via a detuner, such as a detuning circuitry. The detuning circuitry may comprise one or more switches, such as transistors and/or diodes, for changing an inductance of the electromagnetic positioning coil. To detune the electromagnetic positioning coil, the inductance may be reduced. In one or more examples herein, the electromagnetic positioning coil may be short-circuited during normal use, such as when the electromagnetic positioning coil is not active. The short-circuit may be opened to activate the electromagnetic positioning coil.

In one or more examples herein, a similar detune circuit may be applied on the communication channel, such as to the communication interface.

In one or more examples herein, the electronic implant can communicate a signal indicative of an interaction parameter, such as a charging parameter and/or a communication parameter. The signal may be communicated upon the electronic implant detecting that the accessory device is present. The signal may be communicated via a communication interface of the electronic implant to one or more of the bearer of the electronic implant, the accessory device, or a user equipment of the bearer of the electronic implant. The user equipment may be one or more of a smart phone, a smart watch, a tablet, and a computer. In one or more examples herein, the signal indicative of the interaction parameter is continuously or periodically communicated. In one or more examples herein, the signal can be communicated upon the charging of the electronic implant starting, such as when the transmitting inductive charging coil comes close to the receiving inductive charging coil. In one or more example methods, the interaction parameter is a charging quality. The charging quality may be communicated as a percentage of a maximum charging quality. The communication interface may be different from the electromagnetic positioning coil.

In one or more examples herein, the signal indicative of the interaction parameter, such as the charging parameter, is communicated to the accessory device. The accessory device may be configured to communicate the quality of the interaction, such as the quality of charging, to the bearer of the electronic implant. The accessory device may communicate the quality of interaction acoustically, such as by emitting a sound, such as a beep, and/or visually, such as by emitting a light, and/or tactilely, such as by vibrating.

In one or more examples herein, the electronic implant and/or the accessory device sends a signal indicative of the quality of interaction to a user device, such as a user device associated with the bearer of the implant or with an operator of the accessory device. The user equipment may be configured to display, for example via an application installed on the user equipment, the quality of interaction, such as the charging quality.

This allows the bearer of the electronic implant to monitor the quality of interaction, such as the quality of charging, and to manually adjust the position of the accessory device, such as the charging device, in case of failure of the automatic positioning and/or alignment using the electromagnetic positioning coil.

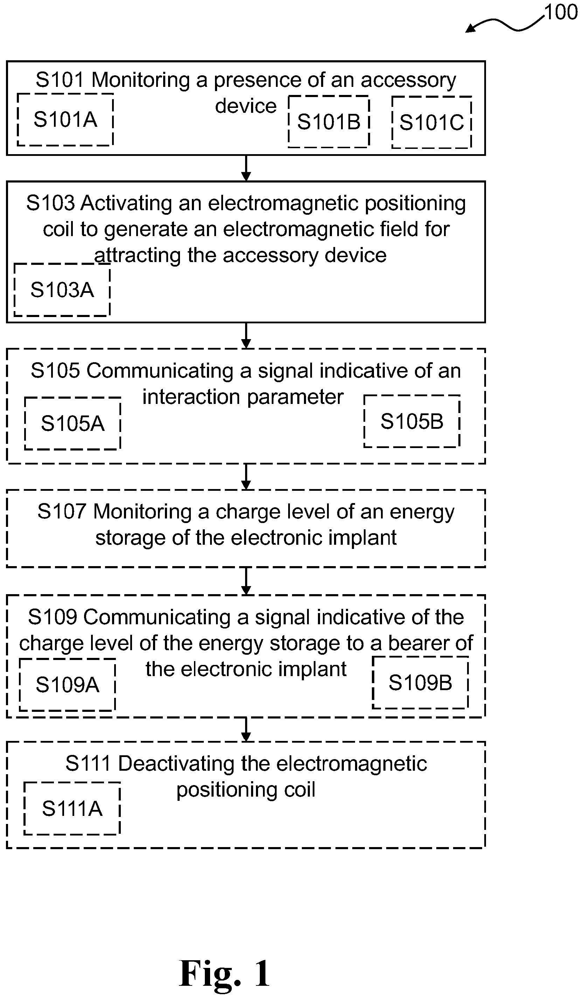

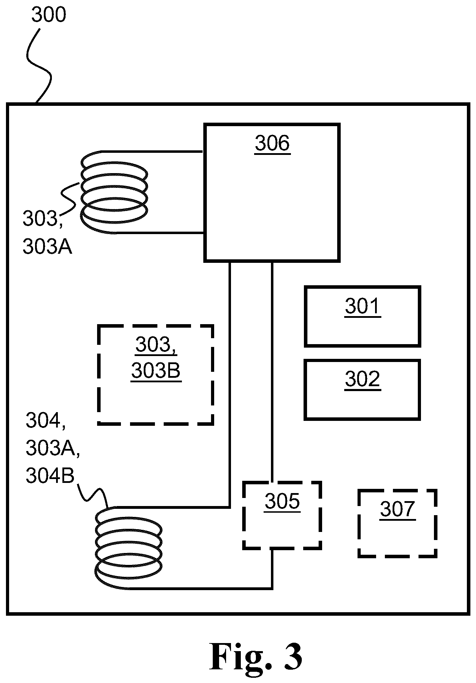

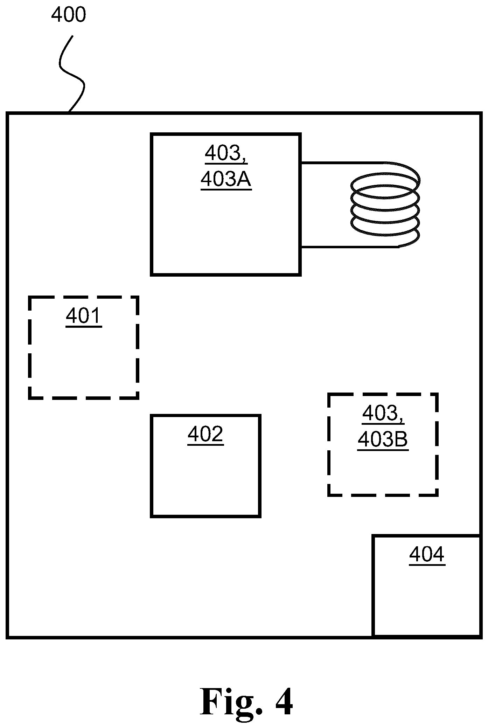

Fig. 1 shows a flow diagram of an example method 100, performed by an electronic implant according to the disclosure, for aligning an accessory device with the electronic implant. The electronic implant comprises an interface for interacting with the accessory device, processing circuitry and an electromagnetic positioning coil. The interface for interacting with the accessory device may be one or more of a communication interface and an inductive charging coil for charging an energy storage, such as a battery, of the electronic implant. The electromagnetic positioning coil may be a coil acting as, such as having the properties of an electromagnet, when being energized. Aligning the accessory device can herein be seen as positioning and orientating the accessory device in relation to the electronic implant. The electronic implant is the electronic implant disclosed herein, such as the electronic implant 300 of Fig. 3 and/or Fig. 4.

The method 100 comprises monitoring S101 a presence of the accessory device. The accessory device may, in one or more examples, be a communication device and the interface for interacting with the accessory device may be a communication interface. The communication interface may for example be a wireless interface for communicating with the accessory device. Monitoring the presence of the accessory device can herein be seen as observing over time whether an accessory device is present.

In one or more example methods, the accessory device is a wireless charging device and the interface for interacting with the accessory device is an inductive charging coil. The inductive charging coil may for example be an inductive charging coil for wireless charging of the electronic implant. The accessory device may in one or more example methods, be both a communication device and a charging device comprising both a communication interface and the inductive charging coil. The inductive charging coil of the electronic implant may herein be referred to as a receiving charging coil.

In one or more example methods, such as when the accessory device comprises an inductive charging coil, monitoring S101 comprises monitoring S101A a magnetic field change at the electronic implant. In one or more example methods, the presence of the accessory device is detected upon a magnetic switch comprised in the electronic implant being activated by an electromagnetic field, such as by an electromagnetic field generated by the accessory device. The electromagnetic field may for example be generated by a charging coil comprised in the accessory device. In other words, the presence of the accessory device may be monitored by monitoring an activation and/or deactivation of the magnetic switch.

In one or more example methods, monitoring S101 comprises monitoring S101B a charging parameter, such as one or more of a current, a voltage, a power, and a charging quality, generated in the first wireless charging interface, such as in the inductive charging coil or the CPT coupling coupler of the electronic implant. In one or more example methods, the accessory device is determined to be present when the charging parameter generated in the inductive charging coil is equal to or above a charging threshold. The charging threshold may be one or more of a charging current threshold, a charging voltage threshold, a charging power threshold, and/or a charging quality threshold.

In one or more example methods, monitoring S101 the presence of the accessory device may comprise monitoring S101C for an activation signal received by the electronic implant via the communication interface. The activation signal may for example be received from the accessory device or from a user equipment associated with the user of the implant.

The method 100 comprises, upon determining that the accessory device is present, activating S103 the electromagnetic positioning coil to generate an electromagnetic field for aligning the accessory device. The magnetic field is configured to attract the accessory device, such as a magnetic field generated by a permanent magnet and/or an electromagnetic positioning coil of the accessory device. The activation may be continuous or non-continuous. The created electromagnetic field is configured to attract and align a magnet comprised in the accessory device. Aligning can herein be seen as positioning and/or orientating the accessory device in relation to the electronic implant. Hence, activating S103 comprises activating the electromagnetic positioning coil to generate an electromagnetic field for positioning and/or orientating the accessory device in relation to the electronic implant. Positioning and/or orientating the accessory device in relation to the electronic implant can herein be seen as positioning and/or orientating a component of the accessory device with a corresponding component of the electronic implant, such as positioning and/or orientating a transmitting charging coil of the accessory device with a receiving charging coil of the electronic implant, and/or positioning and/or orientating a communication interface of the of the accessory device with a communication interface of the electronic implant. The activation of the electromagnetic positioning coil may be continuous or non-continuous. The activation of the electromagnetic positioning coil may for example be based on the monitored charging parameter, such as one or more of the monitored current, voltage, power, and charging quality. The charging quality may be indicative of a quality of the connection between the charging coils of the electronic implant and the accessory device. The charging quality may for example be indicated and/or determined as a rate of a charging current generated in the first wireless charging interface, such as in the inductive charging coil or the CPT coupling coupler, of the electronic implant.

In one or more example methods, activating S103 comprises energizing the electromagnetic positioning coil. By energizing the electromagnetic positioning coil, such as feeding a current through the electromagnetic positioning coil, the electromagnetic field can be generated by the electromagnetic positioning coil.

In one or more example electronic implants, the electronic implant may comprise a detuner, such as a detuning circuitry, for detuning the electromagnetic positioning coil. Detuning can herein be seen as bringing the circuit resonance frequency, such as the resonance frequency of the electromagnetic positioning coil, out of range from a central frequency of another system, such as for example an MRI acquisition system. An example MRI acquisition central frequency may be 127.728 MHz for 3T proton MRI. The detuner may be an electromagnetic detuning unit, for detuning, such as decoupling, the electromagnetic positioning coil from electro-magnetic energy emitted during Magnetic Resonance Imaging (MRI) Radio Frequency (RF) pulses. By detuning the electromagnetic positioning coil, the coil can be prevented from swinging in response to the MRI RF pulses, which otherwise could lead to the electromagnetic positioning coil creating in the MRI. By detuning the electromagnetic positioning coil, a magnetic field of the electromagnetic positioning coil may be switched off. In one or more example methods, activating S103 comprises deactivating S103A the detuner for detuning the electromagnetic positioning coil. By deactivating the detuner the magnetic field of the electromagnetic positioning coil can be activated.

The detuner may for example be configured for detuning unwanted coupling of frequencies typically used for MRI acquisition, such as at 1, 1.5, and 3 Tesla. In one or more example electronic implants, the detuner, such as the detuning circuitry, may be realized by active or passive detuning.

The passive detuning may be realized by a passive detuning circuitry. The passive detuning circuitry may comprise a plurality of diodes, such as Schottky diodes, configured for shortening the coil inductance of the electromagnetic positioning coil.

The active detuning may be realized, by using active elements to shorten the inductance of the electromagnetic positioning coil. The active elements may comprise one or more pin-diodes that are configured to be switched in order to short-circuit the inductance of the electromagnetic positioning coil. However, other active switching methods may also be used in order to detune the electromagnetic positioning coil. In one or more example electronic implants, the electromagnetic positioning coil may be detuned by default. In other words, as long as the electromagnetic positioning coil is not active, such as is not energized, the electromagnetic positioning coil of the electronic implant is in a detuned state. In other words, as long as the coil is not active the coil is short-circuited. The active element, such as a transistor, diode, general-purpose input/output (GPIO), may be configured to deactivate the short-circuit or detuning of the electromagnetic positioning coil when the electromagnetic positioning coil is to be activated. Thereby, the electromagnetic positioning coil is detuned by default and cannot interact with resonance-frequencies of an MRI acquisition system.

In one or more example methods, the method comprises, upon determining that the accessory device is present, communicating S105 a signal indicative of an interaction parameter, such as indicative of the charging parameter generated in the first wireless charging interface of the electronic implant and/or the communication parameter, to a bearer of the electronic implant. The signal may be communicated via the interaction interface, such as via the communication interface. The interaction interface is different from the electromagnetic positioning coil.

In one or more example methods, communicating S105 comprises sending S105A, to the accessory device, the signal indicative of an interaction parameter. The interaction parameter may be one or more of a communication parameter, and the charging parameter generated in the first wireless charging interface. The communication parameter may be indicative of a signal quality, such as a bitrate, bandwidth, signal strength, such as a Received Signal Strength Indicator (RSSI), a signal to noise ratio (SNR), a Q-Factor, and/or a Residual Sum of Squares (RSS), of the signal communicated via the communication interface.

In one or more example methods, the signal indicative of the interaction parameter comprises an indication instructing the accessory device to emit a signal indicative of the interaction parameter, such as of the communication parameter and/or the charging parameter, to the bearer of the electronic implant. In one or more example methods, the signal is indicative of the charging parameter and comprises an indication instructing the accessory device to emit a signal indicative of the charging parameter, such as of one or more charging parameters, to the bearer of the electronic implant. In one or more example methods, the signal indicative of the interaction parameter may be indicative of a remaining time until the electronic implant, such as the energy storage of the electronic implant, is fully charged.

In one or more example methods, the signal indicative of the charging parameter generated in the first wireless charging interface comprises an indication instructing the accessory device, such as the wireless charging device, to adjust a charging parameter, such as one or more charging parameter, of the wireless charging device.

In one or more example methods, communicating S105 comprises sending S105B, to a user equipment of the bearer of the electronic implant, a signal instructing the user equipment to generate an output indicative of the charging parameter generated in the first wireless charging interface. In one or more example methods, the signal instructing the user equipment to generate an output indicative of the charging parameter generated in the first wireless charging interface is sent via the accessory device. The signal may for example instruct the user equipment to generate an output indicative of one or more of a current, a voltage, a power, and a charging quality, generated in the first wireless charging interface. The generated output may be one or more of visual, acoustic, and tactile. In other words, the signal may instruct the user equipment to display a message on a screen, and/or emit an audio signal indicative of the charging parameter, and/or provide tactile feedback, such as output a tactile signal. The tactile signal may for example be a vibration provided to the bearer of the implant via the user equipment.

In one or more example methods, the charging parameter is indicative of an alignment of the accessory device, such as whether the implant is in an alignment mode. In one or more example methods, the charging parameter is indicative of a frequency bandwidth, such as a frequency bandwidth of the voltage and/or current generated in the charging coil. In one or more example methods, the charging parameter is indicative of a current amplitude, such as a current amplitude in the charging coil. In one or more example methods, the charging parameter is indicative of a phase alignment. The phase alignment can be determined based on a phase-angle measurement. A phase delay between an output voltage and a current after a DC conversion in the wireless charging coil is a measure for distance of the first and the second wireless charging coils. The phase alignment may be determined using a processor circuitry, such as a Digital Signal Processor (DSP) controller.

In one or more example methods, the method comprises monitoring S107 a charge level, such as an energy level, of an energy storage of the electronic implant. Monitoring the charge level can herein be seen as observing and checking the progress of the charging of the energy storage.

In one or more example methods, upon determining that the charge level is equal to or above a charge threshold, communicating S109 a signal indicative of the charge level of the energy storage to a bearer of the electronic implant.

In one or more example methods, communicating S109 comprises sending S109A, to the accessory device, such as to one or more accessory devices, a signal indicative of the charge level of the energy storage.

In one or more example methods, the signal indicative of the charge level of the energy storage comprises an indication instructing the accessory device to emit a signal indicative of the charge level of the energy storage to the bearer.

In one or more example methods, the signal indicative of the charge level of the energy storage comprises an indication instructing the accessory device to adjust the charging parameter, such as adjusting one or more of an amplitude, a frequency, and an impedance, of the first wireless charging interface, such as in the inductive charging coil or the CPT coupling coupler. In one or more example methods, the signal indicative of the charge level may instruct the accessory device to stop the wireless charging of the electronic implant.

In one or more example methods, communicating S109 comprises sending S109B, to a user equipment associated with the bearer of the electronic implant, a signal instructing the user equipment to generate an output indicative of the charge level of the energy storage. The generated output indicative of the charge level may be one or more of a visual output, an acoustic output, and a tactile output. In other words, the signal may instruct the user equipment to display a message indicative of the charge level on a screen, and/or emit an audio signal indicative of the charge level, and/or provide tactile feedback indicative of the charge level, such as output a tactile signal indicative of the charge level. The output indicative of the charge level may for example indicate, such as to the bearer of the electronic implant, that the energy storage of the electronic implant has been fully charged and/or that charging has been completed.

In one or more example methods, the method comprises, upon determining that the accessory device is not present, deactivating S111 the electromagnetic positioning coil. By deactivating the electromagnetic positioning coil when the accessory device is not present, the electromagnetic positioning coil does not generate an electromagnetic field when there is no accessory device to align with the electronic implant. By deactivating the electromagnetic positioning coil in the electronic implant, a radio frequency interaction between the electromagnetic positioning coil and an MRI system can be prevented, so that a risk of injuries and/or other artifacts in the MRI can be reduced. In one or more example methods, the electromagnetic positioning coil may be deactivated by activating the detuner to detune the electromagnetic positioning coil. In other words, in one or more example methods, deactivating S111 comprises activating S111A the detuner.

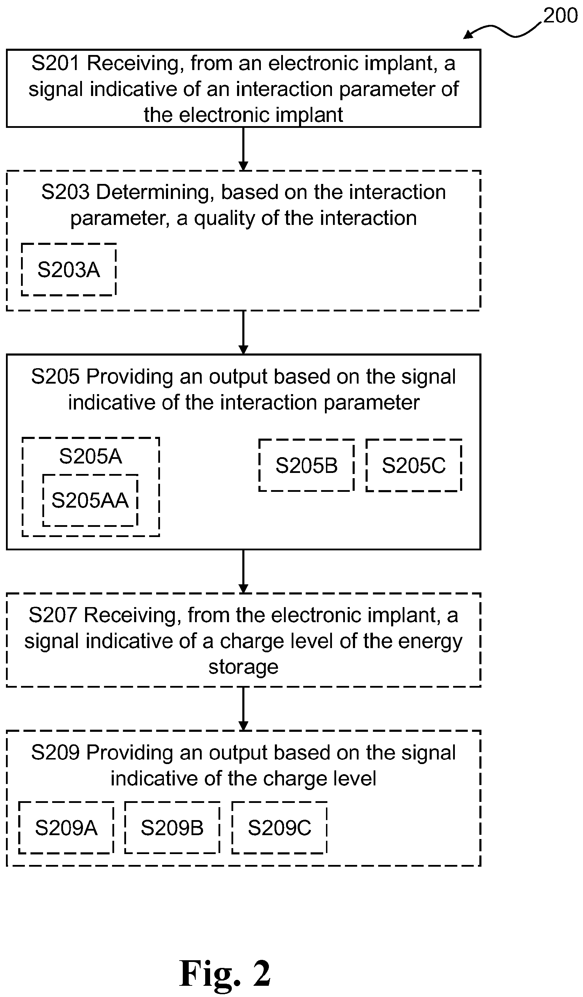

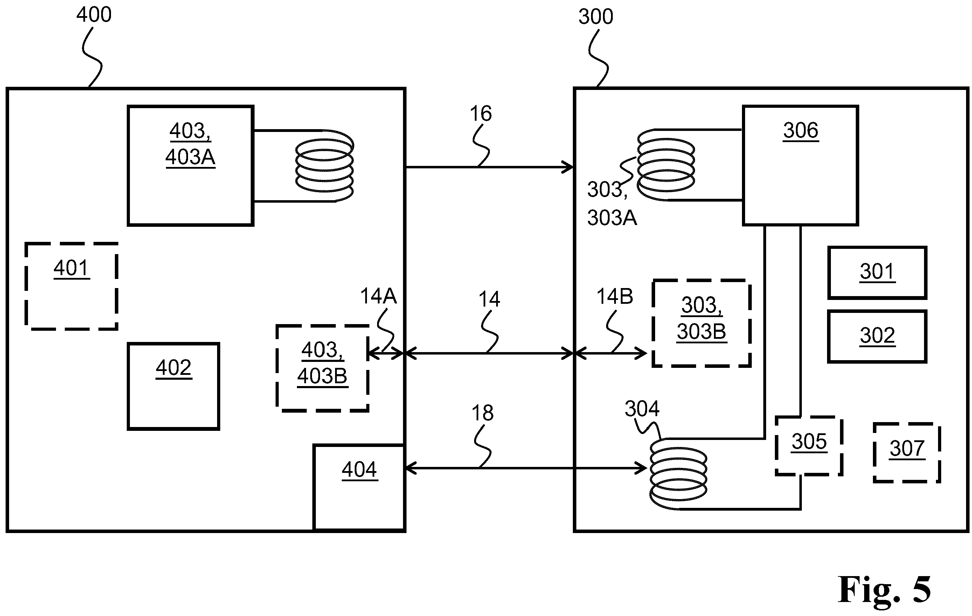

Fig. 2 shows a flow diagram of an example method 200, performed in an accessory device according to the disclosure, for aligning the accessory device with an electronic implant, such as the electronic implant according to this disclosure. The accessory device comprises an interface for interacting with the electronic implant, processing circuitry and a positioning device. The interface for interacting with the accessory device may be one or more of a communication interface for communicating with the electronic implant and a second wireless charging interface, such as an inductive charging coil or a CPT coupling coupler, for wireless charging of the electronic implant. The positioning device may be a magnetic device, such as a permanent magnet or an electromagnetic positioning coil, or a material having magnetic properties. The positioning device may be configured to align with and/or attract to a magnetic field generated by for example the electromagnetic positioning coil of the electronic implant. The accessory device is the accessory device disclosed herein, such as the accessory device 400 of Fig. 4 and/or Fig. 5.

In one or more example methods, the accessory device may output an interaction signal, such as a magnetic or electric field generated in the second wireless charging interface, and/or a communication signal transmitted using the communication interface. Outputting the interaction signal may enable the electronic implant to detect a presence of the accessory device.

The method 200 comprises receiving S201, from the electronic implant, a signal indicative of an interaction parameter. In one or more example methods, the interaction parameter is a communication parameter. The communication parameter may be indicative of a signal quality, such as a bitrate, bandwidth, signal strength, such as a Received Signal Strength Indicator (RSSI), a signal to noise ratio (SNR), a Q-Factor, and/or an RSS, of the signal communicated via the communication interface.

In one or more example methods, the interaction parameter is a charging parameter generated in a first wireless charging interface, such as an inductive charging coil or a CPT coupling coupler, of the electronic implant, such as generated in the first wireless charging interface of the electronic implant by the second wireless charging interface of the accessory device. The second wireless charging interface of the accessory device may herein be referred to as a transmitting wireless interface. The second wireless charging interface of the electronic implant may herein be referred to as a receiving wireless charging interface. The charging parameter may indicate that the inductive charging coil in the electronic implant receives a charge from the accessory device, such as from the transmitting wireless charging interface. In one or more example methods, the signal indicative of the interaction parameter comprises an indication instructing the accessory device to emit a signal indicative of the interaction parameter, such as of the communication parameter and/or the charging parameter, to the bearer of the electronic implant. In one or more example methods, the signal is indicative of the charging parameter and comprises an indication instructing the accessory device to emit a signal indicative of the charging parameter, such as of one or more charging parameters, to the bearer of the electronic implant.

In one or more example methods, the method comprises determining S203, based on the interaction parameter, a quality of the interaction between the accessory device and the electronic implant. In one or more example methods, such as when the interaction parameter is the charging parameter, the method comprises determining S203A, based on the charging parameter, a quality of charging. In one or more example methods, such as when the interaction parameter is the communication parameter, the method comprises determining S203B, based on the communication parameter, a quality of communication.

The method comprises providing S205 an output based on the signal indicative of the interaction parameter. In one or more example methods, providing the output is performed based on the determined quality of interaction, such as the quality of charging and/or the quality of communication. For example, upon the quality of interaction being below an interaction threshold, the accessory device may provide an output indicating that the quality of interaction is reduced and/or that the accessory device is incorrectly aligned. The quality of interaction being reduced can herein be seen as the quality being lower than what it could be in a perfect interaction scenario. In one or more examples, such as upon the quality of interaction being equal to or above the interaction threshold, the accessory device may provide an output indicating that the quality of interaction is good and/or that the accessory device is correctly aligned.

In one or more example methods, providing S205 the output comprises emitting S205A a signal indicative of the interaction parameter to the bearer of the electronic implant, such as to a user equipment associated with the bearer of the electronic implant. In one or more example methods, such as when the interaction parameter is a charging parameter, providing S205 the output comprises emitting S205AA a signal indicative of the rate of a charging current to the bearer of the electronic implant, such as to a user equipment associated with the bearer of the electronic implant. In one or more example methods, the signal indicative of the interaction parameter, such as the charging parameter generated in the first wireless charging interface of the electronic implant, comprises an indication instructing the accessory device to emit a signal indicative of the rate of the charging current to the bearer of the electronic implant. In one or more example methods, emitting S205AA is based on, such as is performed in response to, the indication instructing the wireless charging device to emit the signal.

In one or more example methods, the positioning device is the electromagnetic positioning coil and providing S205 the output comprises activating S205C the electromagnetic positioning coil. In one or more example methods, activating S205C comprises energizing the electromagnetic positioning coil, such as feeding a current through the electromagnetic positioning coil. By energizing the electromagnetic positioning coil in the accessory device, the magnetic field generated by the electromagnetic positioning coil can be attracted by the magnetic field generated in the electronic implant, such as by the electromagnetic positioning coil of the electronic implant.

In one or more example methods, providing S205 the output comprises adjusting S205B the interaction parameter, such as the charging parameter and/or the communication parameter, of the accessory device based on the received signal indicative of the interaction parameter. The interaction parameter may be adjusted by adjusting one or more configuration parameters of the interaction interface, such as the charging interface and/or the communication interface, of the accessory device. In one or more example methods, adjusting the interaction parameter comprises adjusting a charging parameter of the transmitting charging coil, for example based on a received signal indicative of the charging parameter generated in the first wireless charging interface of the electronic implant, such as in the receiving wireless charging interface.

In one or more example methods, the signal indicative of the interaction parameter, such as the signal indicative of the charging parameter generated in the first wireless charging interface of the electronic implant, comprises an indication instructing the wireless charging device to adjust an interaction parameter, such as one or more charging parameters, of the accessory device. Adjusting S205B may, in one or more example methods, be based on, such as may be performed in response to, the indication instructing the wireless charging device to adjust the interaction parameter, such as the charging parameter.

In one or more example methods, the method comprises receiving S207, from the electronic implant, a signal indicative of the charge level of the energy storage. In one or more example methods, the signal indicative of the charge level of the energy storage comprises an indication instructing the accessory device to emit a signal indicative of the charge level. In one or more example methods, the signal indicative of the charge level of the energy storage comprises an indication instructing the wireless charging device to adjust a charging parameter, such as to stop the wireless charging.

In one or more example methods, the method comprises providing S209 an output based on the signal indicative of the charge level. The output may be indicative of the charge level of the energy storage. The output may be indicative of one or more of charging being in progress, charging being completed, energy storage being fully charged, and a percentage of charge of the energy storage. The output may, in one or more example methods, be provided, such as transmitted, to the bearer of the electronic implant, such as to a user equipment associated with the bearer.

In one or more example methods, providing S209 comprises emitting S209A a signal indicative of the charge level. The emitted signal may be one or more of a visual signal, an acoustic signal, and/or a tactile signal.

In one or more example methods, emitting S209A is based on the indication instructing the accessory device to emit the signal indicative of the charge level. In other words, the signal indicative of the charge level of the energy storage may be emitted in response to receiving the indication instructing the accessory device to emit the signal indicative of the charge level.

In one or more example methods, providing S209 comprises adjusting S209B the charging parameter of the accessory device based on the signal indicative of the charge level.

In one or more example methods, the adjusting S209B is based on, such as is performed in response to the indication instructing the wireless charging device to adjust the charging parameter.

In one or more example methods, providing S209 comprises deactivating S209C the electromagnetic positioning coil and/or the second wireless charging interface based on the signal indicative of the charge level. For example, upon the charging being completed, such as upon the energy storage being fully charged, the second wireless charging interface of the accessory device may be deactivated to stop an energy transfer to the electronic implant and/or the electromagnetic positioning coil of the accessory device may be deactivated to release the accessory device from the electronic implant.

Fig. 3 shows a block diagram of an example electronic implant 300 according to the disclosure. The electronic implant 300 comprises memory circuitry 301, processor circuitry 302, an interaction interface 303, such as a wireless charging interface 303A and/or a communication interface 303B, an energy storage 306, and an, such as one or more, electromagnetic positioning coil(s) 304. In one or more example electronic devices, the electronic device may comprise a plurality of electromagnetic positioning coils, such as a first electromagnetic positioning coil 304A, and a second electromagnetic positioning coil 304B. The electromagnetic positioning coil, and/or the plurality of electromagnetic positioning coils, may be comprised in an electromagnetic positioning unit. By using a plurality of electromagnetic positioning coils, a rotation of the accessory device in relation to the electronic implant may be controlled in addition to the positioning and orientation. In one or more example electronic implants, the first electromagnetic charging coil 304A is a Helmholtz coil and the second electromagnetic charging coil 304B is a Saddle coil. The wireless charging interface 300A may be an inductive charging coil configured to generate a current when the inductive charging coil is exposed to a magnetic field, or a CPT coupling coupler configured to generate a current when the coupling coupler is exposed to an electric field. The communication interface 300B may, in one or more example electronic implants, be a wireless communication interface for communicating with an accessory device, such as the accessory device 400 disclosed herein. The electronic implant may, in one or more examples, comprise a detuner 305, such as a detuning circuitry, for detuning the electromagnetic positioning coil 304.

The electronic implant 300 may be configured to perform any of the methods disclosed in Fig. 1. In other words, the electronic implant 300 may be configured for aligning an accessory device with the electronic implant 300.

The electronic implant 300 is configured to monitor, for example using the processor circuitry 302 and/or the interaction interface 303, 303A, 303B, a presence of the accessory device. In one or more examples, the electronic implant 300 may be configured to monitor the presence of the accessory device 400 by being configured to monitor a magnetic field change at the electronic implant. The electronic implant 300 may be configured to determine that the accessory device is present when a charging current in the wireless charging interface 303A is above a charging current threshold. In one or more examples, the electronic implant is configured to monitor the magnetic field change by monitoring a change of current in the inductive charging coil. In one or more examples, the electronic implant 300 may comprise a magnetic switch 307 being configured to be activated when a magnetic field is present. The electronic implant 300 may be configured to determine that the accessory device 400 is present when the magnetic switch 307 has been activated. In other words, the electronic implant 300 can be configured to monitor the presence of the accessory device 400 by monitoring an activation and/or deactivation of the magnetic switch 307.

In one or more example electronic implants, the electronic implant 300 is configured to monitor (for example using the processor circuitry 302, and/or the interaction interface 303, such as the charging interface 303A) a charging parameter, such as one or more of a current, a voltage, a power, and a charging quality, generated in the wireless charging interface 303A, such as in the inductive charging coil or the CPT coupling coupler of the electronic implant.

The electronic implant 300 is configured to, upon determining that the accessory device is present, activate, for example using the processor circuitry 302 and/or the energy storage 306, the electromagnetic positioning coil to generate an electromagnetic field for aligning the accessory device with the electronic implant. The wireless device 300 may, in one or more examples, be configured to activate the electromagnetic positioning coil, by being configured to deactivate (for example using the processor circuitry 302) the detuner 305.

In one or more example electronic implants, the electronic implant is configured to, upon determining that the accessory device is present, communicate (for example using the communication interface 303B) a signal indicative of an interaction parameter, such as indicative of the charging parameter generated in the wireless charging interface 303A of the electronic implant and/or the communication parameter, to a bearer of the electronic implant.

In one or more example electronic implants, the electronic implant is configured to monitor (for example using the processor circuitry 302) a charge level, such as an energy level, of an energy storage of the electronic implant. Monitoring the charge level can herein be seen as observing and checking the progress of the charging of the energy storage 306.

In one or more example electronic implants, the electronic implant is configured to, upon determining that the charge level is equal to or above a charge threshold, communicate (for example using the communication interface 303B) a signal indicative of the charge level of the energy storage to a bearer of the electronic implant.

The communication interface 303 is configured for wireless communications via a wireless communication system, such as via one or more of Bluetooth, Wi-Fi, Near-Field Communication (NFC), Medical Implant Communication Service (MICS), and Industrial-Scientific-Medical (ISM), or a mobile communication system, such as a 3^{rd} Generation Partnership Program (3GPP) system.

The electronic implant 300 is optionally configured to perform any of the operations disclosed in Fig. 1 (such as any one or more of S101, S101A, S101B, S103, S103A, S105, S105A, S105B, S107, S109, S109A, S109B, S111, S111A). The operations of the electronic implant 300 may be embodied in the form of executable logic routines (for example, lines of code, software programs, etc.) that are stored on a non-transitory computer readable medium (for example, memory circuitry 301) and are executed by processor circuitry 302).

Furthermore, the operations of the wireless device 300 may be considered a method that the wireless device 300 is configured to carry out. Also, while the described functions and operations may be implemented in software, such functionality may also be carried out via dedicated hardware or firmware, or some combination of hardware, firmware and/or software.

Memory circuitry 301 may be one or more of a buffer, a flash memory, a hard drive, a removable media, a volatile memory, a non-volatile memory, a random access memory (RAM), or other suitable device. **In** a typical arrangement, memory circuitry 301 may include a non-volatile memory for long term data storage and a volatile memory that functions as system memory for processor circuitry 302. Memory circuitry 301 may exchange data with processor circuitry 302 over a data bus. Control lines and an address bus between memory circuitry 301 and processor circuitry 302 also may be present (not shown in Fig. 3). The memory circuitry 301 is considered a non-transitory computer readable medium.

Memory circuitry 301 may be configured to store information (such as information indicative of the interaction parameter, such as the communication parameter and/or the charging parameter, and/or the charge level) in a part of the memory.

Fig. 4 shows a block diagram of an example accessory device 400 according to the disclosure. The accessory device 400 comprises, processor circuitry 402, and an interaction interface 403 for interacting with the electronic implant, such as a charging interface 403A and/or a communication interface 403B, and a positioning device 404. In one or more examples, the accessory device 400 comprises a memory circuitry 401. The positioning device is configured to align with, and/or attract to, a magnetic field generated by the positioning coil(s) 304 of the electronic implant 300. The positioning device 404 may be one or more of a permanent magnet and an electromagnetic positioning coil. The wireless charging interface 403A may be an inductive charging coil, such as an emitting charging coil, or a CPT coupling coupler, such as an emitting CPT coupling coupler. The accessory device 400 may be configured to perform any of the methods disclosed in Fig. 2. In other words, the accessory device 400 may be configured aligning the accessory device with an electronic implant, such as with the electronic implant 300 disclosed herein. The components 401, 402, 403, 403A, 403B, 404 of the accessory device 400 may be arranged in one or more housings, such as may be arranged in the same housing or may be distributed into different housings.

The accessory device 400 is configured to interact with the electronic implant, such as the electronic implant disclosed herein, using the interaction interface 403, such as the charging interface 403A and/or the communication interface 403B.

In one or more examples, the accessory device 400 may be configured to generate a magnetic field (for example using the second wireless charging interface 403A, such as the inductive charging coil or the CPT coupling coupler), which can be detected by a first wireless charging interface 303A, such as the inductive charging coil or the CPT coupling coupler, in the electronic implant.

The accessory device 400 is configured to receive (for example using the communication interface 303B), from the electronic implant, a signal indicative of an interaction parameter, such as indicative of a communication parameter and/or a charging parameter.

The accessory device 400 is configured to determine, based on the received interaction parameter, a quality of the interaction between the accessory device and the electronic implant. In one or more examples, the accessory device 400 is configured to determine, based on the charging parameter, a quality of charging. In one or more examples, the accessory device 400 is configured to determine, based on the communication parameter, a quality of communication.

In one or more examples, the accessory device 400 is configured to provide an output (for example using the communication interface 303B) based on the signal indicative of the interaction parameter. In one or more examples, the accessory device 400 is configured to provide the output based on the determined quality of interaction, such as the quality of charging and/or the quality of communication. For example, the accessory device may be configured, to upon the quality of interaction being below an interaction threshold, provide an output indicating that the quality of interaction is reduced and/or that the accessory device is incorrectly aligned.

In one or more examples, the accessory device 400 is configured to emit (for example using the communication interface 303B) a signal indicative of the interaction parameter to a bearer of the electronic implant, such as to a user equipment associated with the bearer of the electronic implant. In one or more examples, the accessory device 400 is configured to emit (for example using the communication interface 303B) a signal indicative of the rate of a charging current to the bearer of the electronic implant, such as to a user equipment associated with the bearer of the electronic implant.

In one or more examples, the accessory device 400 is configured to activate the electromagnetic positioning coil. In one or more examples, the accessory device 400 is configured to activate (for example using the processor circuitry 402) the electromagnetic positioning coil 404 by energizing the electromagnetic positioning coil, such as feeding a current through the electromagnetic positioning coil.

In one or more examples, the accessory device 400 is configured to adjust (for example using the processor circuitry 402) the interaction parameter, such as the charging parameter and/or the communication parameter, of the accessory device, such as based on the received signal indicative of the interaction parameter.

In one or more examples, the accessory device 400 is configured to receive, for example using the communication interface 403B, a signal indicative of the charge level of the energy storage.

In one or more examples, the accessory device 400 is configured to provide, such as emit and/or transmit (for example using the communication interface 403B), an output based on the signal indicative of the charge level.

In one or more examples, the accessory device 400 is configured to adjust (for example using the processor circuitry 402) the charging parameter of the accessory device, such as adjusting one or more of an amplitude, a frequency, and an impedance, of the wireless charging interface 403A, based on the signal indicative of the charge level.

In one or more examples, the accessory device 400 is configured to deactivate the electromagnetic positioning coil and/or the wireless charging interface based on the signal indicative of the charge level, such as upon the signal indicative of the charge level being equal to or above a charge threshold.

The communication interface 403 is configured for wireless communications via a wireless communication system, such as via one or more of Bluetooth, Wi-Fi, Near-Field Communication (NFC), Medical Implant Communication Service (MICS), and Industrial-Scientific-Medical (ISM), or a mobile communication system, such as a 3^{rd} Generation Partnership Program (3GPP) system.

The accessory device 400 is configured to send, for example, via the wireless interface 403, to a UE, information indicative of one or more interaction parameter.

Processor circuitry 402 is optionally configured to perform any of the operations disclosed in Fig. 2 (such as any one or more of S201, S203, S203A, S205, S205A, S205AA, S205B, S205C, S207, S209, S209A, S209B, S209C). The operations of the accessory device 400 may be embodied in the form of executable logic routines (for example, lines of code, software programs, etc.) that are stored on a non-transitory computer readable medium (for example, memory circuitry 401) and are executed by processor circuitry 402.

Furthermore, the operations of the accessory device 400 may be considered a method that the accessory device 400 is configured to carry out. Also, while the described functions and operations may be implemented in software, such functionality may also be carried out via dedicated hardware or firmware, or some combination of hardware, firmware and/or software.

Memory circuitry 401 may be one or more of a buffer, a flash memory, a hard drive, a removable media, a volatile memory, a non-volatile memory, a random access memory (RAM), or other suitable device. In a typical arrangement, memory circuitry 401 may include a non-volatile memory for long term data storage and a volatile memory that functions as system memory for processor circuitry 402. Memory circuitry 401 may exchange data with processor circuitry 402 over a data bus. Control lines and an address bus between memory circuitry 401 and processor circuitry 402 also may be present (not shown in Fig. 4). Memory circuitry 401 is considered a non-transitory computer readable medium.

Memory circuitry 401 may be configured to store information (such as information indicative of the interaction parameter, such as the communication parameter and/or the charging parameter, and/or the charge level) in a part of the memory.

Fig. 5 discloses a wireless interaction system, such as a wireless charging system, comprising the electronic implant 300 disclosed in Fig. 3 and the accessory device 400 disclosed in Fig. 4. The electronic implant 300 and the accessory device comprise respective interaction interfaces 303, 403, such as wireless charging interfaces 303A, 403A configured to establish an energy transfer connection 16 between the accessory device 400 and the electronic implant 300, and/or a communication interfaces 303B, 403B configured to establish a communication channel 14, 14A, 14B between the electronic implant and the accessory device 400.

To ensure that the quality of interaction, such as the charging quality and/or the communication quality is sufficient, the electronic implant 300 can activate the electromagnetic positioning coil 304, for example upon detecting that the accessory device 400 is present, such as in the vicinity of the electronic implant. By activating the electromagnetic positioning coil 304 a magnetic field is generated which creates a magnetic force 18 for attracting and aligning the positioning device 404 of the accessory device 400. The electromagnetic positioning coil 304 and the positioning device 404 may be configured so that, when the positioning device 404 of the accessory device 400 has been attracted and aligned with the electromagnetic positioning coil 304 of the electronic implant 300, the interaction interface 303 of the electronic implant, such as the charging interface 303, and/or the communication interface 303B, is aligned, such as overlap, with the corresponding interaction interface 303 403 of the accessory device 400, such as the charging interface 403A, and/or the communication interface. In one or more example methods, the interaction interfaces 303, 303A, 303B of the electronic implant 300 and the interaction interfaces 403, 403A, 403B accessory device 400 are arranged in the electronic implant 300 and the accessory device 400, respectively, such that the interaction interfaces 303, 303A, 303B of the electronic implant 300 and the interaction interfaces 403, 403A, 403B accessory device 400 are aligned when the positioning device 404 of the accessory device 400 has been attracted and aligned with the electromagnetic positioning coil 304 of the electronic implant 300.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

**It** may be appreciated that Figures 1-5 comprise some circuitries or operations which are illustrated with a solid line and some circuitries, components, features, or operations which are illustrated with a dashed line. Circuitries or operations which are comprised in a solid line are circuitries, components, features, or operations which are comprised in the broadest example. Circuitries, components, features, or operations which are comprised in a dashed line are examples which may be comprised in, or a part of, or are further circuitries, components, features, or operations which may be taken in addition to circuitries, components, features, or operations of the solid line examples. **It** should be appreciated that these operations need not be performed in order presented. Furthermore, it should be appreciated that not all of the operations need to be performed. The example operations may be performed in any order and in any combination. **It** should be appreciated that these operations need not be performed in order presented. Circuitries, components, features, or operations which are comprised in a dashed line may be considered optional.

Other operations that are not described herein can be incorporated in the example operations. For example, one or more additional operations can be performed before, after, simultaneously, or between any of the described operations.

Certain features discussed above as separate implementations can also be implemented in combination as a single implementation. Conversely, features described as a single implementation can also be implemented in multiple implementations separately or in any suitable sub-combination. Moreover, although features may be described above as acting in certain combinations, one or more features from a claimed combination can, in some cases, be excised from the combination, and the combination may be claimed as any sub-combination or variation of any sub-combination
It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the examples may be implemented at least in part by means of both hardware and software, and that several "means", "units" or "devices" may be represented by the same item of hardware.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1 % of, and within less than or equal to 0.01% of the stated amount. **If** the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount.

The various example methods, devices, and systems described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program circuitries may include routines, programs, objects, components, data structures, etc. that perform specified tasks or implement specific abstract data types. Computer-executable instructions, associated data structures, and program circuitries represent examples of program code for executing steps of the methods disclosed herein. The particular sequence of such executable instructions or associated data structures represents examples of corresponding acts for implementing the functions described in such steps or processes.

The present invention is set out in the claims that follow.

## Claims

1. A method, performed by an electronic implant, for aligning an accessory device with the electronic implant, wherein the electronic implant comprises an interface for interacting with the accessory device, processing circuitry, and one or more electromagnetic positioning coils, the method comprising:
- monitoring (S101) a presence of the accessory device, and
- upon determining that the accessory device is present, energizing (S103) the one or more electromagnetic positioning coils by feeding a current through the one or more electromagnetic positioning coils to generate a magnetic field for aligning the accessory device.

2. The method according to claim 1, wherein the generated magnetic field is for positioning the accessory device in relation to the electronic implant.

3. The method according to claim 1 or 2, wherein the generated magnetic field is for orientating the accessory device in relation to the electronic implant.

4. The method according to any of the previous claims, wherein the electronic implant comprises an energy storage and/or wherein the interface of the electronic implant comprises a wireless charging interface, and wherein an energy used to energize the one or more electromagnetic positioning coils is supplied by one of the energy storage and the charging interface.

5. The method according to claim 4, wherein the accessory device is a wireless charging device and the interface for interacting with the accessory device is a wireless charging interface, and wherein the energy used to energize the one or more electromagnetic positioning coils is supplied by the wireless charging interface during a charging procedure.

6. The method according to claim 5, wherein the wireless charging interface comprises an inductive charging coil different from the one or more positioning coils or a capacitive power transfer (CPT) coupling coupler, and wherein the energy used to energize the one or more electromagnetic positioning coils is supplied by one of the inductive charging coil and the CPT coupling coupler during a charging procedure.

7. An electronic implant (300) comprising an interface (303) for interacting with an accessory device (400), an electromagnetic positioning coil (304), processor circuitry (302), and an energy storage (306), wherein the electronic implant is configured to perform any of the methods according to any of claims 1-6.

8. A computer readable storage medium storing one or more programs, the one or more programs comprising instructions, which when executed by an electronic implant according to claim 7 cause the electronic implant to perform any of the methods of claims 1-6.

9. A method, performed by an accessory device, for aligning the accessory device with an electronic implant, wherein the accessory device comprises an interface for interacting with the electronic implant, processing circuitry, and a positioning device being an electromagnetic positioning coil, and wherein the electronic implant is configured to generate a first magnetic field, the method comprising:
- receiving (S201), from the electronic implant, a signal indicative of an interaction parameter, and
- providing (S205), in response to the signal indicative of the interaction parameter, an output comprising energizing (S205C) the electromagnetic positioning coil of the positioning device to generate a second magnetic field for aligning the positioning device with and/or attracting the positioning device to the first magnetic field.

10. The method according to claim 9, wherein energizing (S205C) the electromagnetic positioning coil comprises feeding a current through the electromagnetic positioning coil.

11. The method according to any of claims 9-10, wherein the interaction parameter is a charging parameter generated in a wireless charging interface of the implant, wherein the method comprises determining (S203), based on the charging parameter, a quality of charging; and wherein providing (S205) the output is performed based on the determined quality of charging.

12. The method according to any one of the claims 9-11, wherein the method comprises:
- receiving (S207), from the electronic implant, a signal indicative of the charge level of the energy storage, and
- providing (S209) an output based on the signal indicative of the charge level.

13. An accessory device (400) comprising processing circuitry (402), a positioning device (404), and an interaction interface (403), wherein the accessory device is configured to perform any of the methods according to any of claims 9-11.

14. A computer readable storage medium storing one or more programs, the one or more programs comprising instructions, which when executed by an accessory device according to claim 13 cause the accessory device to perform any of the methods of claims 9-11.

15. A wireless interaction system for interacting with an electronic implant, the wireless interaction system comprising the accessory device according to claim 13 and the electronic implant according to claim 7.

## Patentansprüche

1. Verfahren, durchgeführt durch das elektronische Implantat, zur Ausrichtung einer Zubehörvorrichtung mit einem elektronischen Implantat, wobei das elektronische Implantat eine Schnittstelle zur Interaktion mit der Zubehörvorrichtung, eine Verarbeitungsschaltung und eine oder mehrere elektromagnetische Positionierspulen umfasst, wobei das Verfahren umfasst:
- Überwachen (S101) auf Vorhandensein der Zubehörvorrichtung, und
- nach Bestimmen, dass die Zubehörvorrichtung vorhanden ist, Erregen (S103) der einen oder mehreren elektromagnetischen Positionierspulen durch Zuführen eines Stroms durch die eine oder mehreren elektromagnetischen Positionierspulen, um ein Magnetfeld zur Ausrichtung der Zubehörvorrichtung zu erzeugen.

2. Verfahren nach Anspruch 1, wobei das erzeugte Magnetfeld dazu dient, die Zubehörvorrichtung in Bezug auf das elektronische Implantat zu positionieren.

3. Verfahren nach Anspruch 1 oder 2, wobei das erzeugte Magnetfeld dazu dient, die Zubehörvorrichtung in Bezug auf das elektronische Implantat auszurichten.

4. Verfahren nach einem der vorstehenden Ansprüche, wobei das elektronische Implantat einen Energiespeicher umfasst und/oder wobei die Schnittstelle des elektronischen Implantats eine drahtlose Ladeschnittstelle umfasst und wobei die Energie, die zum Erregen der einen oder mehreren elektromagnetischen Positionierspulen verwendet wird, von einem vom Energiespeicher oder von der Ladeschnittstelle zugeführt wird.

5. Verfahren nach Anspruch 4, wobei die Zubehörvorrichtung eine drahtlose Ladevorrichtung ist und die Schnittstelle zur Interaktion mit der Zubehörvorrichtung eine drahtlose Ladeschnittstelle ist und wobei die Energie, die zum Erregen der einen oder mehreren elektromagnetischen Positionierspulen verwendet wird, während eines Ladevorgangs von der drahtlosen Ladeschnittstelle zugeführt wird.

6. Verfahren nach Anspruch 5, wobei die drahtlose Ladeschnittstelle eine induktive Ladespule umfasst, die sich von der einen oder den mehreren Positionierspulen oder einem kapazitiven Energieübertragungs- (CPT) Kopplungskoppler unterscheidet, und wobei die Energie, die zum Erregen der einen oder mehreren elektromagnetischen Positionierspulen verwendet wird, während eines Ladevorgangs von der induktiven Ladespule oder dem CPT-Kopplungskoppler zugeführt wird.

7. Elektronisches Implantat (300), das eine Schnittstelle (303) zur Interaktion mit einer Zubehörvorrichtung (400), eine elektromagnetische Positionierspule (304), eine Prozessorschaltung (302) und einen Energiespeicher (306) umfasst, wobei das elektronische Implantat so konfiguriert ist, dass es eines der Verfahren nach einem der Ansprüche 1-6 durchführt.

8. Computerlesbares Speichermedium, das ein oder mehrere Programme speichert, wobei das eine oder die mehreren Programme Anweisungen umfassen, die, wenn sie von einem elektronischen Implantat nach Anspruch 7 ausgeführt werden, das elektronische Implantat veranlassen, eines der Verfahren nach Ansprüchen 1-6 durchzuführen.

9. Verfahren, durchgeführt durch die Zubehörvorrichtung, zur Ausrichtung einer Zubehörvorrichtung mit einem elektronischen Implantat, wobei die Zubehörvorrichtung eine Schnittstelle zur Interaktion mit dem elektronischen Implantat, eine Verarbeitungsschaltung und eine Positioniervorrichtung in Form einer elektromagnetischen Positionierspule umfasst und wobei das elektronische Implantat so konfiguriert ist, dass es ein erstes Magnetfeld erzeugt, wobei das Verfahren umfasst:
- Empfangen (S201), vom elektronischen Implantat, eines Signals, das einen Interaktionsparameter anzeigt, und
- Bereitstellen (S205), als Reaktion auf das Signal, das den Interaktionsparameter anzeigt, eines Ausgangs, umfassend Erregen (S205C) der elektromagnetischen Positionierspule der Positioniervorrichtung, um ein zweites Magnetfeld zum Ausrichten der Positioniervorrichtung mit und/oder Anziehen der Positioniervorrichtung zum ersten Magnetfeld zu erzeugen.

10. Verfahren nach Anspruch 9, wobei Erregen (S205C) der elektromagnetischen Positionierspule Einspeisen eines Stroms durch die elektromagnetische Positionierspule umfasst.

11. Verfahren nach einem der Ansprüche 9-10, wobei der Interaktionsparameter ein in einer drahtlosen Ladeschnittstelle des Implantats erzeugter Ladeparameter ist, wobei das Verfahren Bestimmen (S203) einer Ladequalität auf Basis des Ladeparameters umfasst; und wobei Bereitstellen (S205) des Ausgangs auf Basis der bestimmten Ladequalität erfolgt.

12. Verfahren nach einem der Ansprüche 9-11, wobei das Verfahren umfasst:
- Empfangen (S207), vom elektronischen Implantat, eines Signals, das den Ladezustand des Energiespeichers anzeigt, und
- Bereitstellen (S209) eines Ausgangs basierend auf dem Signal, das den Ladezustand anzeigt.

13. Zubehörvorrichtung (400), die eine Verarbeitungsschaltung (402), eine Positioniervorrichtung (404) und eine Interaktionsschnittstelle (403) umfasst, wobei die Zubehörvorrichtung so konfiguriert ist, dass sie eines der Verfahren nach einem der Ansprüche 9-11 durchführt.

14. Computerlesbares Speichermedium, das ein oder mehrere Programme speichert, wobei das eine oder die mehreren Programme Anweisungen umfassen, die, wenn sie von einer Zubehörvorrichtung nach Anspruch 13 ausgeführt werden, die Zubehörvorrichtung veranlassen, eines der Verfahren nach Ansprüchen 9-11 durchzuführen.

15. Drahtloses Interaktionssystem zur Interaktion mit einem elektronischen Implantat, wobei das drahtlose Interaktionssystem die Zubehörvorrichtung nach Anspruch 13 und das elektronische Implantat nach Anspruch 7 umfasst.

## Revendications

1. Procédé, réalisé par un implant électronique, permettant d'aligner un dispositif accessoire avec l'implant électronique, dans lequel l'implant électronique comprend une interface permettant d'interagir avec le dispositif accessoire, l'ensemble de circuits de traitement et une ou plusieurs bobines de positionnement électromagnétiques, le procédé comprenant :
- la surveillance (S101) de la présence du dispositif accessoire, et
- après avoir déterminé que le dispositif accessoire est présent, l'alimentation (S103) des une ou plusieurs bobines de positionnement électromagnétiques en faisant passer un courant à travers les une ou plusieurs bobines de positionnement électromagnétiques afin de générer un champ magnétique pour aligner le dispositif accessoire.

2. Procédé selon la revendication 1, dans lequel le champ magnétique généré est destiné à positionner le dispositif accessoire par rapport à l'implant électronique.

3. Procédé selon la revendication 1 ou 2, dans lequel le champ magnétique généré est destiné à orienter le dispositif accessoire par rapport à l'implant électronique.

4. Procédé selon l'une quelconque des revendications précédentes, dans lequel l'implant électronique comprend un système de stockage d'énergie et/ou dans lequel l'interface de l'implant électronique comprend une interface de charge sans fil, et dans lequel l'énergie utilisée pour alimenter les une ou plusieurs bobines de positionnement électromagnétiques est fournie par l'un des éléments parmi le système de stockage d'énergie et l'interface de charge.

5. Procédé selon la revendication 4, dans lequel le dispositif accessoire est un dispositif de charge sans fil et l'interface permettant d'interagir avec le dispositif accessoire est une interface de charge sans fil, et dans lequel l'énergie utilisée pour alimenter les une ou plusieurs bobines de positionnement électromagnétiques est fournie par l'interface de charge sans fil au cours d'une procédure de charge.

6. Procédé selon la revendication 5, dans lequel l'interface de charge sans fil comprend une bobine de charge inductive différente des une ou plusieurs bobines de positionnement ou d'un coupleur de couplage de transfert d'énergie capacitive (CPT), et dans lequel l'énergie utilisée pour alimenter les une ou plusieurs bobines de positionnement électromagnétiques est fournie par l'un des éléments parmi la bobine de charge inductive et le coupleur de couplage de CPT au cours d'une procédure de charge.

7. Implant électronique (300) comprenant une interface (303) permettant d'interagir avec un dispositif accessoire (400), une bobine de positionnement électromagnétique (304), l'ensemble de circuits de traitement (302) et un système de stockage d'énergie (306), dans lequel l'implant électronique est configuré pour réaliser l'un des procédés selon l'une quelconque des revendications 1-6.

8. Support de stockage lisible par ordinateur contenant un ou plusieurs programmes, les un ou plusieurs programmes comprenant des instructions qui, lorsqu'elles sont exécutées par un implant électronique selon la revendication 7, amènent l'implant électronique à réaliser l'un des procédés selon les revendications 1-6.

9. Procédé, réalisé par un dispositif accessoire, permettant d'aligner le dispositif accessoire avec un implant électronique, dans lequel le dispositif accessoire comprend une interface permettant d'interagir avec l'implant électronique, l'ensemble de circuits de traitement et un dispositif de positionnement étant une bobine de positionnement électromagnétique, et dans lequel l'implant électronique est configuré de manière à générer un premier champ magnétique, le procédé comprenant :
- la réception (S201), à partir de l'implant électronique, d'un signal indiquant un paramètre d'interaction, et
- la fourniture (S205), en réponse au signal indiquant le paramètre d'interaction, d'une sortie comprenant l'alimentation (S205C) de la bobine de positionnement électromagnétique du dispositif de positionnement afin de générer un second champ magnétique pour aligner le dispositif de positionnement avec et/ou attirer le dispositif de positionnement vers le premier champ magnétique.

10. Procédé selon la revendication 9, dans lequel l'alimentation (S205C) de la bobine de positionnement électromagnétique comprend le fait de faire passer un courant à travers la bobine de positionnement électromagnétique.

11. Procédé selon l'une quelconque des revendications 9-10, dans lequel le paramètre d'interaction est un paramètre de charge généré dans une interface de charge sans fil de l'implant, dans lequel le procédé comprend la détermination (S203), sur la base du paramètre de charge, d'une qualité de charge ; et dans lequel la fourniture (S205) de la sortie est réalisée sur la base de la qualité de charge déterminée.

12. Procédé selon l'une quelconque des revendications 9-11, dans lequel le procédé comprend :
- la réception (S207), à partir de l'implant électronique, d'un signal indiquant le niveau de charge du système de stockage d'énergie, et
- la fourniture (S209) d'une sortie sur la base du signal indiquant le niveau de charge.

13. Dispositif accessoire (400) comprenant l'ensemble de circuits de traitement (402), un dispositif de positionnement (404) et une interface d'interaction (403), dans lequel le dispositif accessoire est configuré pour réaliser l'un des procédés selon l'une quelconque des revendications 9-11.

14. Support de stockage lisible par ordinateur contenant un ou plusieurs programmes, les un ou plusieurs programmes comprenant des instructions qui, lorsqu'elles sont exécutées par un dispositif accessoire selon la revendication 13, amènent le dispositif accessoire à réaliser l'un des procédés selon les revendications 9-11.

15. Système d'interaction sans fil permettant d'interagir avec un implant électronique, le système d'interaction sans fil comprenant le dispositif accessoire selon la revendication 13 et l'implant électronique selon la revendication 7.
